# EUROPEAN PATENT APPLICATION

(11) **EP 4 725 930 A1**
(43) Date of publication of application: **15.04.2026**
(21) Application number: 24819326.0
(22) Date of filing: 04.06.2024
(51) Int. Cl.: C07C 17/367, C07C 21/18, C07C 21/185, C07C 23/06, C08J 11/28

(54) **FLUOROMONOMER PRODUCTION METHOD**

(30) Priority: 06.06.2023 JP 2023093289
(71) Applicant: AGC INC., Chiyoda-ku, Tokyo 1008405 (JP)
(72) Inventor: FUJITA, Tomoyuki, Tokyo 100-8405 (JP); SAKAI, Takayuki, Tokyo 100-8405 (JP)
(74) Representative: Müller-Boré & Partner Patentanwälte PartG mbB
(86) International application number: PCT/JP2024/020390
(87) International publication number: WO 2024/253097

(57) **Abstract**

Provided is a method for producing a fluoromonomer with an excellent conversion rate of fluoropolymer.

The method for producing a fluoromonomer according to the present invention includes heating a fluoropolymer in the presence of an organic sulfur compound to obtain a fluoromonomer by thermal decomposition of the fluoropolymer.

## Description

### TECHNICAL FIELD

The present invention relates to a method for producing a fluoromonomer.

### BACKGROUND ART

Recycling of fluoropolymers such as polytetrafluoroethylene has long been desired. For example, Patent Document 1 discloses a process for producing a fluoroolefin by thermal decomposition of a fluoropolymer in the presence of steam with the use of a rotary kiln.

### PRIOR ART DOCUMENTS

### PATENT DOCUMENTS

Patent Document 1: Japanese Patent No. 4010300

### DISCLOSURE OF INVENTION

### TECHNICAL PROBLEM

For production of a fluoromonomer from a fluoropolymer, further improvement in the proportion of the fluoropolymer converted to the fluoromonomer (that is, the conversion rate) has been demanded.

The present inventors have studied the production of fluoromonomers from fluoropolymers by the process disclosed in Patent Document 1, and found that there is room for improvement in the conversion rate of fluoropolymers.

In view of the above-mentioned problem, the present invention has been made to provide a method for producing a fluoromonomer with a high conversion rate of fluoropolymer.

### SOLUTION TO PROBLEM

As a result of intensive studies made on the above-mentioned problem, the present inventors have found that the conversion rate of a fluoropolymer is improved when the fluoropolymer is heated in the presence of an organic sulfur compound, and thus have accomplished the present invention.

That is, the present inventors have found the following solutions to the above-mentioned problem.
[1] A method for producing a fluoromonomer, comprising heating a fluoropolymer in the presence of an organic sulfur compound to obtain a fluoromonomer by thermal decomposition of the fluoropolymer.
[2] The method for producing a fluoromonomer according to [1], wherein the organic sulfur compound comprises at least one type selected from the group consisting of polyphenylene sulfide, polyethersulfone and polysulfone.
[3] The method for producing a fluoromonomer according to [1] or [2], wherein the fluoropolymer comprises at least one type selected from the group consisting of polytetrafluoroethylene, a tetrafluoroethylene-based copolymer, polychlorotrifluoroethylene, polyvinylidene fluoride, polyvinyl fluoride, a vinylidene fluoride-hexafluoropropylene copolymer and a perfluoropolyether rubber.
[4] The method for producing a fluoromonomer according to any one of [1] to [3], wherein the amount of the organic sulfur compound used is 2 to 300 parts by mass, taking the amount of the fluoropolymer used as 100 parts by mass.
[5] The method for producing a fluoromonomer according to any one of [1] to [4], wherein the thermal decomposition of the fluoropolymer is carried out using a rotary kiln.
[6] The method for producing a fluoromonomer according to any one of [1] to [5], wherein a heating temperature during the heating of the fluoropolymer is from 300°C to 800°C.

### ADVANTAGEOUS EFFECTS OF INVENTION

According to the present invention, there can be provided a method for producing a fluoromonomer with a high conversion rate of fluoropolymer.

### BRIEF DESCRIPTION OF DRAWINGS

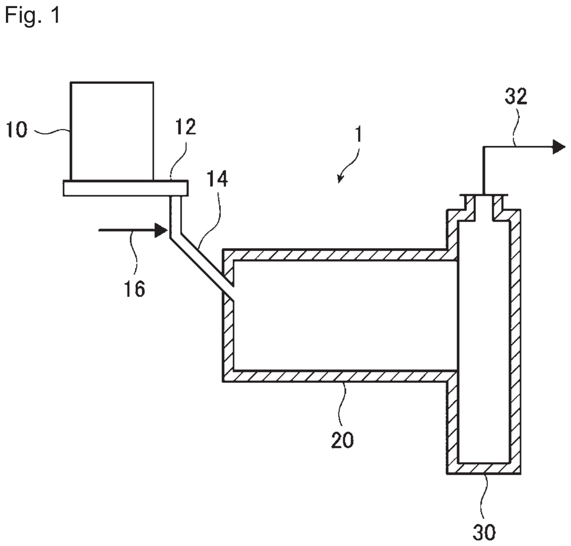

Fig. 1 is a schematic side view illustrating an example of a thermal decomposition device used in a method for producing a fluoromonomer according to the present invention.

### DESCRIPTION OF EMBODIMENTS

The meanings of terms in the present invention are as follows.

A numerical range expressed using "to" means a range including numerical values described before and after "to" as the lower and upper limits.

A "unit" is a generic term for an atomic group derived from one molecule of monomer, which is formed directly by polymerization of the monomer, and an atomic group obtained by chemical conversion of a part of the aforementioned atomic group. A "unit based on a monomer" is hereinafter also simply referred to as a "unit".

### [Method for Producing Fluoromonomer]

A method for producing a fluoromonomer according to the present invention (hereinafter also referred to as the "present production method") includes heating a fluoropolymer in the presence of an organic sulfur compound to conduct thermal decomposition of the fluoropolymer and thereby obtain a fluoromonomer. The present production method is suitable for recycling of fluoropolymers.

The present production method achieves a high conversion rate of fluoropolymers. The detailed reason for this is not clear, but is assumed to be as follows: the heating of a fluoropolymer in the presence of an organic sulfur compound promotes the formation of radicals during thermal decomposition of the fluoropolymer to improve the thermal decomposition efficiency of the fluoropolymer and consequently improve the conversion rate of the fluoropolymer.

Even in the case where the heating temperature for thermal decomposition of the fluoropolymer is set to a relatively low temperature (for example, 300 to 600°C) in the present production method, the thermal decomposition of the fluoropolymer proceeds favorably due to the use of the organic sulfur compound so that the conversion rate of the fluoropolymer can be sufficiently improved.

In the case where a sulfur-containing gas such as hydrogen sulfide is used instead of the organic sulfur compound, there are problems in terms of safety and in view of the requirement for gas introduction equipment.

### <Fluoropolymer>

The fluoropolymer is not particularly limited so far as it is a polymer having fluorine atoms. The fluoropolymer can be a resin or a rubber.

The fluoropolymer may be one obtained from fluoropolymer-containing waste materials etc.

From the viewpoint of obtaining more excellent effects of the present invention, the fluoropolymer preferably includes at least one type selected from the group consisting of polytetrafluoroethylene (hereinafter also referred to as "PTFE"), a tetrafluoroethylene-based copolymer, polychlorotrifluoroethylene (hereinafter also referred to as "PCTFE"), polyvinylidene fluoride (hereinafter also referred to as "PVdF"), polyvinyl fluoride (hereinafter also referred to as "PVF"), a vinylidene fluoride-hexafluoropropylene copolymer (hereinafter also referred to as "FKM") and a perfluoropolyether rubber. The fluoropolymer may be one type alone or a combination of two or more types.

Specific examples of the tetrafluoroethylene-based copolymer include a tetrafluoroethylene-ethylene copolymer (hereinafter also referred to as "ETFE"), a tetrafluoroethylene-hexafluoropropylene copolymer (hereinafter also referred to as "FEP"), a tetrafluoroethylene-propylene copolymer (hereinafter also referred to as "FEPM") and a tetrafluoroethylene-perfluoroalkyl vinyl ether copolymer.

As the tetrafluoroethylene-perfluoroalkyl vinyl ether copolymer, preferred are a copolymer (hereinafter also referred to as "PFA") in which, to the total amount of tetrafluoroethylene units (hereinafter also referred to as "TFE units") and perfluoroalkyl vinyl ether units (hereinafter also referred to as "PAVE units"), the proportion of PAVE units is 0.1 mol% or more and less than 20 mol% and a copolymer (hereinafter also referred to as "FFKM") in which the proportion of PAVE units to the total amount of TFE units and PAVE units is 20 to 70 mol%.

The PFA may be a copolymer consisting only of TFE units and PAVE units or may be a copolymer having, in addition to these units, at least one type of units based on the other monomer. Specific examples of the other monomer include any other fluoromonomer (for example, hexafluoropropylene) and a monomer having an oxygen-containing polar group (for example, 5-norbornene-2,3-dicarboxylic anhydride). The FFKM may be a copolymer consisting only of TFE units and PAVE units or may be a copolymer having, in addition to these units, at least one type of units based on the other monomer. Specific examples of the other monomer include a monomer having a fluorine atom and a nitrile group and a monomer having a fluorine atom and a plurality of vinyl groups.

Although the form of the fluoropolymer used is not particularly limited, the fluoropolymer is preferably in sheet form or particulate form with a view to achieving further improvement in thermal decomposition efficiency. In this case, the size of the fluoropolymer is preferably 1 to 10 mm, more preferably 2 to 5 mm, from the viewpoint of obtaining more excellent effects of the present invention. It is herein assumed that: when the fluoropolymer is in sheet form, the size of the fluoropolymer refers to a sheet thickness; and when the fluoropolymer is particulate form, the size of the fluoropolymer refers to an average particle size on a sieve basis. The thickness can be measured with a thickness gauge or the like, and the particle size can be measured with sieves of respective mesh sizes.

The fluoropolymer may be processed into the above size, in advance, by grinding, cutting or the like.

### <Organic Sulfur Compound>

The organic sulfur compound is an organic compound containing a sulfur atom. The organic sulfur compound may be a polymer compound or a low molecular weight compound, but is preferably a polymer compound.

From the viewpoint of obtaining more excellent effects of the present invention, the organic sulfur compound preferably includes at least one type selected from the group consisting of polyphenylene sulfide (hereinafter also referred to as "PPS"), polyethersulfone (hereinafter also referred to as "PES") and polysulfone (hereinafter also referred to as "PS"), and more preferably includes PPS. The organic sulfur compound may be one type alone or a combination of two or more types.

The PPS can be, for example, a polymer obtained by thermal polycondensation of p-dichlorobenzene and Na₂S.

The PES can be, for example, a polymer obtained by polycondensation of dihalogenodiphenylsulfone and bisphenol S.

The PS can be, for example, a polymer obtained by polycondensation of bisphenol A and 4,4'-dichlorodiphenylsulfone.

Although the form of the organic sulfur compound used is not particularly limited, the organic sulfur compound is preferably in sheet form or particulate form with a view to achieving further improvement in thermal decomposition efficiency. In this case, the size of the organic sulfur compound is preferably 1 to 10 mm, more preferably 2 to 5 mm, from the viewpoint of obtaining more excellent effects of the present invention. It is herein assumed that: when the organic sulfur compound is in sheet form, the size of the organic sulfur compound refers to a sheet thickness; and when the organic sulfur compound is in particulate form, the size of the organic sulfur compound refers to an average particle size on a sieve basis. The thickness can be measured with a thickness gauge or the like, and the particle size can be measured with sieves of respective mesh sizes.

The organic sulfur compound may be processed into the above size, in advance, by grinding, cutting or the like.

The amount of the organic sulfur compound used is preferably 2 to 300 parts by mass, more preferably 3 to 140 parts by mass, still more preferably 5 to 70 parts by mass, taking the amount of the fluoropolymer used as 100 parts by mass.

When the amount of the organic sulfur compound used is 2 parts by mass or more, it is possible to obtain more excellent effects of the present invention. When the amount of the organic sulfur compound used is 300 parts by mass or less, the thermal decomposition efficiency of the fluoropolymer is good relative to the amount of heat applied.

### <Process>

The present production method includes heating the fluoropolymer in the presence of the organic sulfur compound to thermally decompose the fluoropolymer.

The heating temperature of the fluoropolymer is preferably 300°C or higher, more preferably 350°C or higher, still more preferably 400°C or higher, with a view to improving the thermal decomposition efficiency of the fluoropolymer.

**It** is preferred that the heating temperature of the fluoropolymer is lower in view of the design temperature of equipment. More specifically, the heating temperature of the fluoropolymer is preferably 800°C or lower, more preferably 700°C or lower, still more preferably 600°C or lower.

The thermal decomposition of the fluoropolymer can be carried out by means of a known heating device, and is preferably carried out with the use of a rotary kiln from the viewpoint of obtaining more excellent effects of the present invention.

The rotary kiln is a rotary furnace. In the present production method, for example, a device disclosed in Japanese Patent No. 4010300 or a device disclosed in Japanese Patent No. 4357999 can be used.

Fig. 1 is a schematic side view illustrating an example of a thermal decomposition device usable in the present production method, along with a part of its internal structure.

A thermal decomposition device 1 has: a hopper 10 capable of storing therein a raw material (fluoropolymer, organic sulfur compound etc.); a cylindrical rotary kiln 20 into which the raw material is supplied; and a recovery vessel 30 connected to the rotary kiln 20 and into which a thermal decomposition product (fluoromonomer etc.) and residue of the raw material are recovered.

The rotary kiln 20 is equipped with: rotation means (not shown) for rotating the rotary kiln 20; and heating means (not shown) for heating the raw material supplied to the inside of the rotary kiln 20. Further, stirring means may be provided inside the rotary kiln 20 for improvement in the thermal decomposition efficiency of the raw material.

A material constituting an inner wall surface of the rotary kiln 20 is not particularly limited, but preferably contains at least one type selected from the group consisting of stainless steel, nickel and chromium, and may be an alloy of any of these metals.

The raw material supplied and temporarily stored in the hopper 10 is conveyed by a conveyer 12 that is disposed at a position adjacent to the hopper 10, and is supplied to the inside of the rotary kiln 20 via a charging chute 14 that is connected to the rotary kiln 20.

In addition to the raw material, steam (preferably, superheated steam) may be supplied to the charging chute 14 via a stream supply pipe 16. The stream supplied to the charging chute is blown into the rotary kiln 20 via the charging chute. Although the steam supply pipe 16 is connected to the charging chute 14 in the illustrated example of Fig. 1, the thermal decomposition device is not limited to this example. The steam supply pipe 16 may be connected directly to the rotary kiln 20.

The raw material supplied to the inside of the rotary kiln 20 is stirred with rotation of the rotary kiln 20 while being heated by the heating means. With this, the raw material is thermally decomposed within the rotary kiln 20. The resulting thermal decomposition product of the raw material is fed to the recovery vessel 30. A gas component in the thermal decomposition product of the raw material is recovered via a gas recovery pipe 32; and a solid component in the thermal decomposition product of the raw material is recovered from the inside of the recovery vessel 30.

Into the hopper 10, solid substance (e.g. pellets, particles, sheet or the like) containing the fluoropolymer and solid substance containing the organic sulfur compound may be charged separately, or solid substance containing both of the fluoropolymer and the organic sulfur compound may be charged.

### <Fluoromonomer>

Specific examples of the fluoromonomer obtainable by the present production method include tetrafluoroethylene (hereinafter also referred to as "TFE"), hexafluoropropylene (hereinafter also referred to as "HFP"), octafluorocyclobutane (hereinafter also referred to as "c-318"), vinylidene fluoride, viny fluoride, chlorotrifluoroethylene, perfluoroalkyl vinyl ether and a mixture of two or more thereof.

### EXAMPLES

The present invention will be now described in further detail with reference to Examples. Here, Ex. 1 to Ex. 4 correspond to Examples of the present invention; and Ex. 5 corresponds to Comparative Example. It should however be understood that the present invention is by no means restricted to these Examples.

### [Raw Material]

The details of raw materials used in the respective Ex. are shown below.

PTFE (polytetrafluoroethylene): PTFE sheets (manufactured by AS ONE Corporation, thickness: 1 mm) were cut into 5 mm squares and used.

PPS (polyphenylene sulfide): PPS films (manufactured by AS ONE Corporation, thickness: 0.1 mm) were cut into 5 mm squares and used.

PS (polysulfone): PS pellets (manufactured by Kanto Chemical Co., Inc.) were ground and used.

### [Thermal Decomposition Device]

For thermal decomposition of the fluoropolymer in each Ex., the above-described thermal decomposition device of Fig. 1 was used. The details of the rotary kiln in the thermal decomposition device are shown below.
Material constituting inner wall surface of rotary kiln: nickel alloy
Inner size of rotary kiln: diameter: 100 mm, length: 500 mm

### [Composition Analysis of Fluoromonomer]

The composition analysis of a monomer obtained in each Ex. was conducted by gas chromatography. The analysis equipment and analysis conditions used are shown below.
Equipment: Agilent 7890B (manufactured by Agilent Technologies, Inc)
Column: Poraplot-Q (inner diameter: 0.32 mm, length: 25 m, film thickness: 10 µm)
Detector: FID
Column temperature: 100°C
Inlet temperature: 200°C
Detector temperature: 250°C

### [Ex. 1]

The inside of the rotary kiln of the thermal decomposition device was heated to 600°C. After that, thermal decomposition of PTFE was carried out by supplying PPS at 18 g/hr, PTFE at 300 g/hr and steam at 1000 g/hr into the rotary kiln. During the thermal decomposition, the rotation speed of the rotary kiln was set to 5 rpm.

After 2 hours of the above operation, the amount of a solid component recovered from the rotary kiln and the recovery vessel was measured, and the conversion rate of PTFE was calculated from the charged amount. The results are shown in Table 1.

When gas generated during the above operation was analyzed by gas chromatography, TFE, HFP and c-318 were detected. The selectivity of each component is shown in Table 1.

### [Ex. 2 and Ex. 3]

Thermal decomposition of PTFE was carried out in the same manner as in Ex. 1, except that the supply amount of PPS introduced into the rotary kiln was changed to a value shown in Table 1.

The conversion rate of PTFE and the analysis results of gas generated during the operation are shown in Table 1.

### [Ex. 4]

Thermal decomposition of PTFE was carried out in the same manner as in Ex. 2, except that PS was used in place of PPS.

The conversion rate of PTFE and the analysis results of gas generated during the operation are shown in Table 1.

### [Ex. 5]

Thermal decomposition of PTFE was carried out in the same manner as in Ex. 1, except that PPS was not used.

The conversion rate of PTFE and the analysis results of gas generated during the operation are shown in Table 1.

**[Table 1]**

| | | | Ex. 1 | Ex. 2 | Ex. 3 | Ex. 4 | Ex. 5 |
|---|---|---|---|---|---|---|---|
| Supply Amount | PTFE | g/hr | 300 | 300 | 300 | 300 | 300 |
| | PPS | g/hr | 18 | 50 | 150 | | |
| | PS | g/hr | | | | 50 | |
| Heating Temp. of Fluoropolymer | | °C | 600 | 600 | 600 | 600 | 600 |
| Organic Sulfur Compound/ Fluoropolymer | | parts by mass | 6 | 17 | 50 | 17 | 0 |
| Conversion Rate | | % | 92 | 94 | 98 | 75 | 50 |
| Selectivity | TFE | % | 55 | 49 | 40 | 51 | 71 |
| | HFP | % | 25 | 30 | 32 | 22 | 13 |
| | c-318 | % | 9 | 11 | 13 | 7 | 13 |

In Table 1, the term "organic sulfur compound/fluoropolymer" refers to the amount (unit: parts by mass) of the organic sulfur compound used (the total amount of the organic sulfur compound supplied into the rotary kiln) when the amount of the fluoropolymer used (the total amount of the fluoropolymer supplied into the rotary kiln) was taken as 100 parts by mass.

As shown in Table 1, it has been confirmed that the conversion rate of the fluoropolymer is high when the thermal decomposition of the fluoropolymer is carried out in the presence of the organic sulfur compound (Ex. 1 to Ex. 4).

The entire disclosure of Japanese Patent Application No. 2023-093289 filed on June 6, 2023, including its specification, claims, drawings and summary, is incorporated herein by reference in its entirety.

### REFERENCE SYMBOLS

1: Thermal decomposition device
10: Hopper
12: Conveyor
14: Charging chute
16: Steam supply pipe
20: Rotary kiln
30: Recovery vessel
32: Gas recovery pipe

## Claims

1. A method for producing a fluoromonomer, comprising heating a fluoropolymer in the presence of an organic sulfur compound to obtain a fluoromonomer by thermal decomposition of the fluoropolymer.

2. The method for producing a fluoromonomer according to Claim 1, wherein the organic sulfur compound comprises at least one type selected from the group consisting of polyphenylene sulfide, polyethersulfone and polysulfone.

3. The method for producing a fluoromonomer according to Claim 1 or 2, wherein the fluoropolymer comprises at least one type selected from the group consisting of polytetrafluoroethylene, a tetrafluoroethylene-based copolymer, polychlorotrifluoroethylene, polyvinylidene fluoride, polyvinyl fluoride, a vinylidene fluoride-hexafluoropropylene copolymer and a perfluoropolyether rubber.

4. The method for producing a fluoromonomer according to Claim 1 or 2, wherein the amount of the organic sulfur compound used is 2 to 300 parts by mass, taking the amount of the fluoropolymer used as 100 parts by mass.

5. The method for producing a fluoromonomer according to Claim 1 or 2, wherein the thermal decomposition of the fluoropolymer is carried out using a rotary kiln.

6. The method for producing a fluoromonomer according to Claim 1 or 2, wherein a heating temperature during the heating of the fluoropolymer is from 300°C to 800°C.
